# EUROPEAN PATENT APPLICATION

(11) **EP 4 162 932 A1**
(43) Date of publication of application: **12.04.2023**
(21) Application number: 21201384.1
(22) Date of filing: 07.10.2021
(51) Int. Cl.: A61K 31/085, A61K 45/06, A61P 31/14

(54) **ANTIVIRAL HOP CONSTITUENTS**

(71) Applicant: HHV Hallertauer Hopfenveredelungsgesellschaft mbH, 84048 Mainburg (DE)
(72) Inventor: BIENDL, Martin, 84094 Elsendorf (DE)
(74) Representative: Müller-Boré & Partner Patentanwälte PartG mbB

(57) **Abstract**

The present invention relates to compositions comprising xanthohumol for use in a method of treating a Coronavirus infection in a subject.

## Description

The present invention relates to compositions comprising xanthohumol for use in a method of treating a Coronavirus infection in a subject.

Coronaviruses are a group of related RNA viruses that cause diseases in mammals and birds. In humans and birds, they cause respiratory tract infections that can range from mild to lethal. Mild illnesses in humans include some cases of the common cold (which is also caused by other viruses, predominantly rhinoviruses), while more lethal varieties can cause SARS, MERS, and COVID-19.

The coronavirus SARS-CoV-2 has spread worldwide in recent months, causing a worldwide COVID-19 pandemic. Despite intensive protective measures and safety precautions, the virus and also the associated disease COVID-19 continues to spread. This has sometimes devastating health consequences, but also immense social and economic consequences.

Intensive research worldwide has led to the generation and regulatory approval of several suitable vaccines. However, in spite of the ongoing worldwide vaccination campaign, COVID-19 is still prevalent.

Therefore, the search for alternative treatment strategies for Coronavirus infections such as SARS-CoV-2 infection is of particular importance. However, no active substance is yet available that can be used with high efficiency and sufficient tolerability for therapy against Coronaviruses such as SARS-CoV-2.

Accordingly, the technical problem underlying the present invention is the provision of means for the efficient, affordable, and safe treatment of Coronavirus infections such as SARS-CoV-2 infection and the associated COVID-19.

The solution to the above technical problem is achieved by the embodiments characterized in the claims.

In particular, in a first aspect, the present invention relates to a composition comprising xanthohumol for use in a method of preventing or treating a Coronavirus infection in a subject.

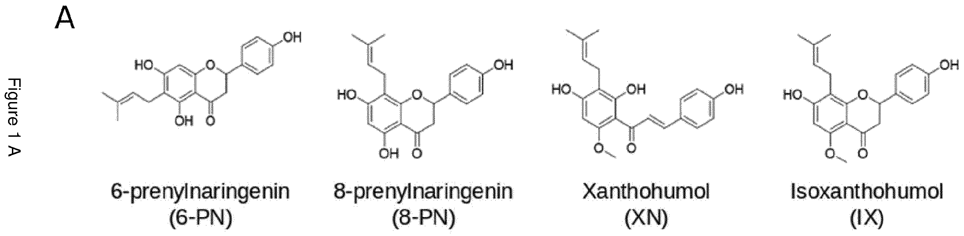

Xanthohumol (XN; Fig. 1 A) is a natural product found in the female inflorescences of *Humulus lupulus,* also known as hops. It is one among four major prenylflavonoids that are typical for hops, the others being 6-prenylnaringenin (6-PN; Fig. 1 A), 8-prenylnaringenin (8-PN; Fig. 1 A), and isoxanthohumol (IX; Fig. 1 A). XN is also found in beer and belongs to a class of compounds that contribute to the bitterness and flavor of hops.

Benefits of XN have been suggested in a number of conditions, including antineoplastic, antiviral, anti-inflammatory and anti-oxidative properties. Thus, XN has been widely used as a natural food supplement, e.g. in the form of hop extracts, for some time. Studies of the safety and pharmacokinetics of XN have shown no adverse effects in humans. Further, XN can be cheaply manufactured from hop extract and, therefore, is readily available on a global scale. Further, processes for the production of synthetic XN are available.

In preferred embodiments, the Coronavirus infection with which is to be treated is a Betacoronavirus, more preferably a Coronavirus that is selected from the group consisting of Betacoronavirus 1 (Bovine Coronavirus, Human coronavirus OC43), Hedgehog coronavirus 1, Human coronavirus HKU1, Middle East respiratory syndrome (MERS)-related coronavirus (MERS-CoV), Murine coronavirus, *Pipistrellus* bat coronavirus HKU5, *Rousettus* bat coronavirus HKU9, Severe acute respiratory syndrome (SARS)-related coronavirus (SARS-CoV, SARS-CoV-2), and *Tylonycteris* bat coronavirus HKU4.

In particularly preferred embodiments, the Coronavirus is MERS-CoV, SARS-CoV or SARS-CoV-2, wherein SARS-CoV-2 is especially preferred.

Further, the subject to be treated in accordance with the present invention is preferably a bird or a mammal, more preferably a mammal, yet more preferably a feline, canine, bovine, swine, equine, camel, murine, mustelid (e.g. a ferret or a mink), or human, and most preferably a human.

In further embodiments, the composition used in the present invention comprises one or more additional agents, selected from the group consisting of polyphenols (e.g. curcumin, resveratrol), antioxidants (e.g. vitamin E, beta-carotene, coenzyme Q10, vitamin C), minerals (e.g. selenium, zinc, iron, cobalt, chromium, copper, iodine, potassium, calcium, manganese, molybdenum), anti-inflammatory agents (e.g. methylene blue (methylthioninium chloride), carprofen, clecoxib, baricitinib, dexamethasone), and antiviral agents (e.g. 5-aminolevulinic acid, abacavir, lamivudine, zidovudine, didanosine, nevirapine, stavudine, lopinavir, ritonavir, tenofovir, emtricitabine, dolutegravir, raltegravir, cabotegravir, cobicistat, rilpivirine, favipiravir, remdesivir, efavirenz, atazanavir, elvitegravir, etravirine, maraviroc, hydroxychloroquine, chloroquine).

In specific embodiments, the method of preventing or treating a Coronavirus infection in a subject in which the composition comprising xanthohumol according to the present invention is used, comprises a step of administering said composition to the subject in a daily dose of 0.05 to 100 mg XN, preferably 0.05 to 50 mg XN, more preferably 0.05 to 20 mg XN, more preferably 0.05 to 10 mg XN, more preferably 0.05 to 5 mg XN, more preferably 0.05 to 2 mg XN, more preferably 0.05 to 1 mg XN, more preferably 0.05 to 0.5 mg XN, more preferably 0.05 to 0.4 mg XN, more preferably 0.05 to 0.35 mg XN, more preferably 0.1 to 0.3 mg, more preferably about 0.25 mg or about 0.125 mg.

In further specific embodiments, the method of preventing or treating a Coronavirus infection in a subject in which the composition comprising xanthohumol according to the present invention is used, comprises a step of administering said composition to the subject in a dose of 0.625 µg to 1250 µg XN per kg body weight of the subject per day (0.625 to 1250 µg/kg/d XN), more preferably 0.625 to 625 µg/kg/d XN, more preferably 0.625 to 250 µg/kg/d XN, more preferably 0.625 to 125 µg/kg/d XN, more preferably 0.625 to 62.5 µg/kg/d XN, more preferably 0.625 to 25 µg/kg/d XN, more preferably 0.625 to 12.5 µg/kg/d XN, more preferably 0.625 to 6.25 µg/kg/d XN, more preferably 0.625 to 5 µg/kg/d XN, more preferably 0.625 to 4.375 µg/kg/d XN, more preferably 1.25 to 3.75 µg/kg/d XN, more preferably about 3.125 µg/kg/d XN or about 1.5625 µg/kg/d XN.

Administration of the compositions described herein can be effected by any method that enables delivery of the compounds to the site of action. These methods include, though are not limited to delivery via enteral routes (including oral, gastric or duodenal feeding tube, rectal suppository and rectal enema), parenteral routes (injection or infusion, including intraarterial, intracardiac, intradermal, intraduodenal, intramedullary, intramuscular, intraosseous, intraperitoneal, intrathecal, intravascular, intravenous, intravitreal, epidural and subcutaneous), inhalational, transdermal, transmucosal, sublingual, buccal and topical (including epicutaneous, dermal, enema, eye drops, ear drops, intranasal, vaginal) administration. Preferably administration of the compositions described herein is effected via the oral route or via the intranasal route. The compositions can be in the form of tablets, capsules, lozenges, sachets, powders, drops, solution, spray, dosage spray, nasal spray, nasal drops, or nasal cream. Further, the compositions can be formulated as nanoparticles or nanoemulsions as known in the art (e.g. via encapsulation with proteins derived from e.g. maize or rice). In specific embodiments, the compositions described herein are in the form of a food supplement or dietary supplement. The compositions can further comprise one or more of pharmaceutically acceptable excipients, diluents, binders, disintegrants, lubricants, glidants, sweetening agents, flavoring agents and colorants.

In a preferred embodiment, the composition of the present invention is for use in a method of treating a Coronavirus infection in a subject.

In a second aspect, the present invention relates to a method of preventing or treating a Coronavirus infection in a subject in need thereof, comprising the step of administering a composition comprising a therapeutically effective amount of xanthohumol to the subject.

In this aspect, all relevant definitions and limitations as defined for the first aspect of the present invention equally apply. In particular, the Coronavirus and subject can be as defined above. The same applies to suitable doses, dosage forms, and/or routes of administration for XN in the prevention or treatment of SARS-CoV-2 infection. Further, the method is preferably a method of treating a Coronavirus infection in a subject in need thereof.

As used herein, the term "comprising"/"comprises" expressly includes the terms "consisting essentially of"/"consists essentially of" and "consisting of"/"consists of", *i.e*., all of said terms are interchangeable with each other herein.

Further, as used herein, the term "about" preferably represents a modifier of the specified value of ± 10%, more preferably ± 10%, ± 8%, ± 6%, ± 5%, ± 4%, ± 3%, ± 2%, ± 1%, or ± 0.5%. Thus, by way of example, the term "about 100" can include the ranges of 90 to 110, 92 to 108, 94 to 106, 95 to 105, 96 to 104, 97 to 103, 98 to 102, 99 to 101, or 99.5 to 100.5.

The present invention identified xanthohumol as an inhibitor of SARS-CoV-2 infection that is characterized by an unexpected and advantageously high efficacy. Thus, the present invention provides highly efficient, affordable, and safe means for the treatment of Coronavirus infections such as SARS-CoV-2 infection and the associated COVID-19 that are readily available worldwide.

Further, the present invention identified one (among possibly several) mechanism of the inhibition of SARS-CoV-2 infection by XN, *i.e*., the inhibition of SARS-CoV-2 Papain-Like Protease (Example 2), which is an enzyme that has homologs in a number of Coronaviruses.

The figures show:

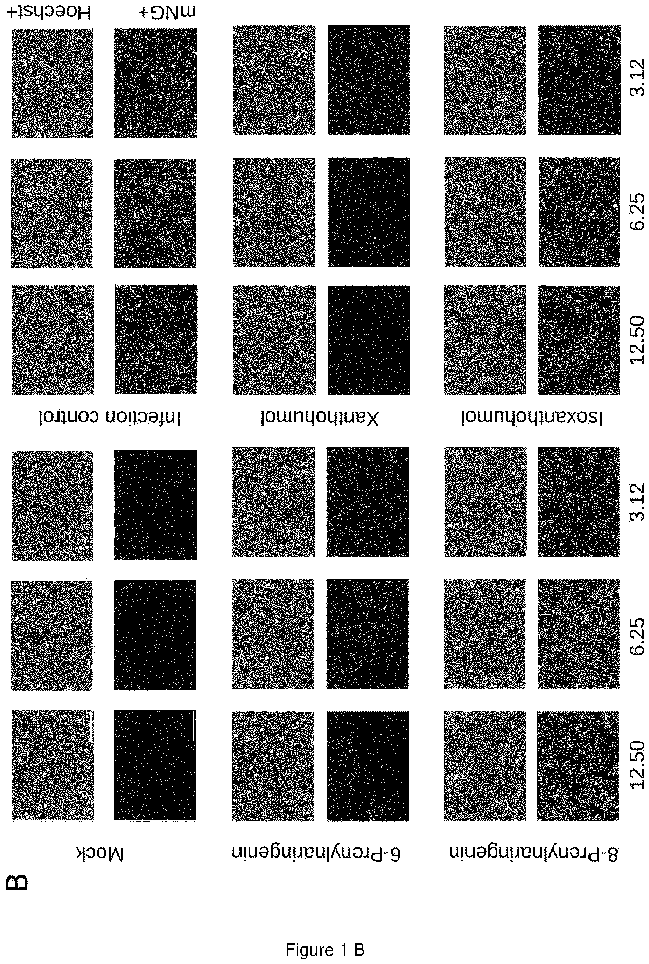

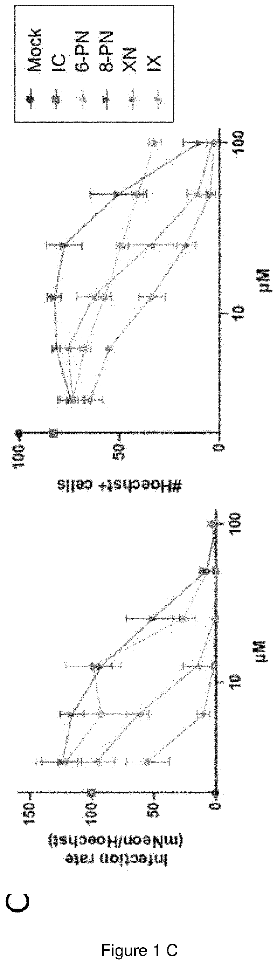

Figure 1: Inhibition of SARS-CoV-2 infection rate by hop compound treatment.
   **A.** Chemical structures of 6-prenylnaringenin (6-PN), 8-prenylnaringenin (8-PN), xanthohumol (XN), and isoxanthohumol (IX). **B.** Microscopical analysis of Caco-2 cells after infection with icSARS-CoV-2-mNG followed by hop compound treatment. Cells were infected with icSARS-CoV-2-mNG or mock-infected. Simultaneously, cells were treated with the indicated compounds in a concentration range from 3.12 to 12.5 µM. 48 h post-infection (hpi), cells were fixed with 2% PFA and stained with Hoechst33342. Representative fluorescence microscopy images, taken at 4-fold magnification. In the upper row of each set, the total amount of cells for each well is shown, as Hoechst+. In the lower row of each set, infected cells are visualized, indicated as mNG1+cells. Scale bar = 1000 µm. **C.** The total number of Caco-2 cells (Hoechst+) remaining after icSARS-CoV-2-mNG infection and treatment with hop compounds for 48 h was plotted against the concentration of the compound (3.12 to 100 µM). The number of infected cells (mNeonGreen+) and the total number of cells (Hoechst+) were analyzed by automated microscopy, and the infection rate was calculated as the ratio of them (left). Effect of hop compounds on the viability of Caco-2 cells (right). Bars represent mean values of three independent experiments +/- SEM.

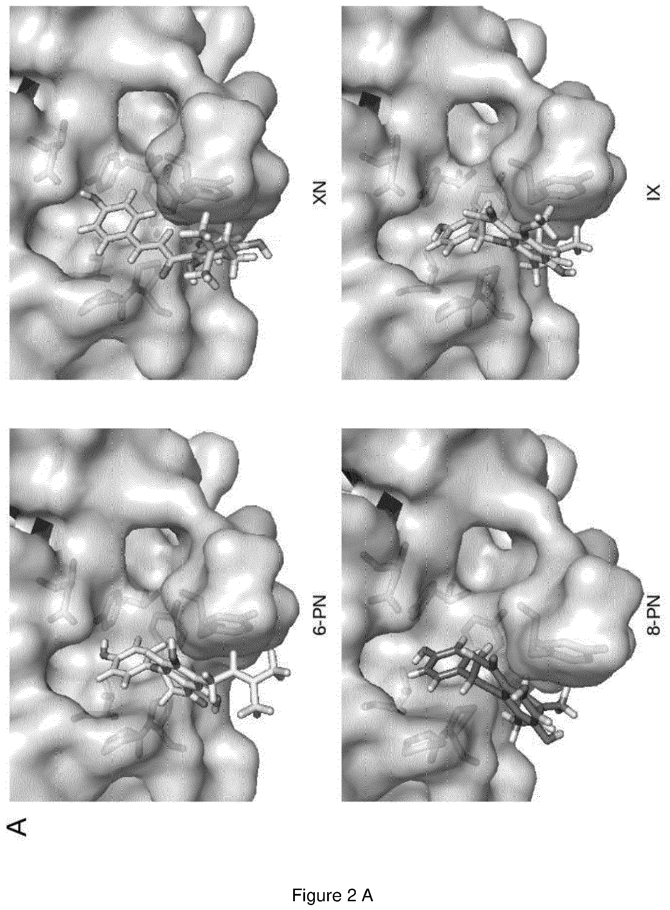

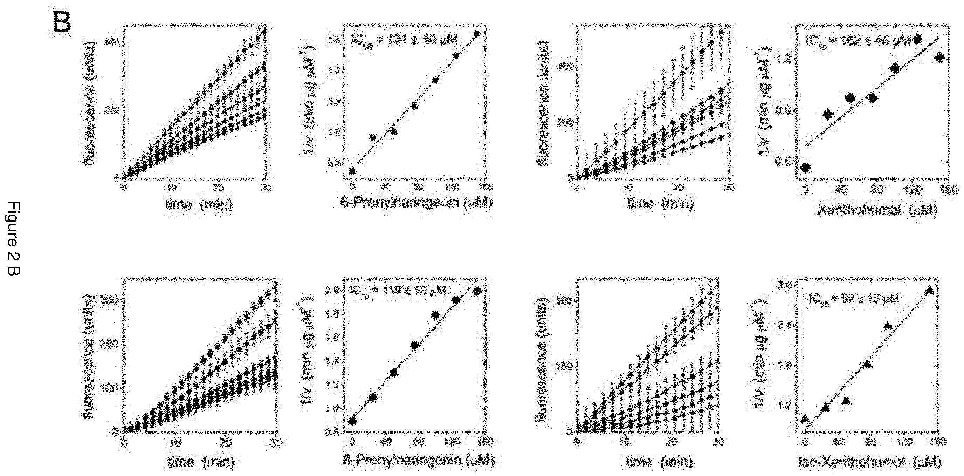

Figure 2: Inhibition of Sars-CoV-2 Papain-Like Protease by different hop compounds.
   **A.** Docking results and proposed binding of hop compounds to the active site of SARS-CoV-2 Papain-Like Protease (PLpro). *In silico* docking analysis of hop compounds fitting into the active site of PLpro (grey) with 6-PN (yellow), 8-PN (violet), XN (green), and IX (turquoise). **B.** Kinetic traces showing the release of fluorophore AMC upon cleavage of a peptide mimicking the C-terminus of human ISG15. The addition of hop compounds 6-PN, 8-PN, XN, and IX differently inhibits the cleavage of the ISG15 peptide as shown by the decrease in the slope of the trace. Traces upon addition of 0 µM, 25 µM, 75 µM, 100 µM, 125 µM, and 150 µM of hop compounds are shown. Reciprocal plots of enzymatic activity versus the concentration of hop compounds are depicted. The IC₅₀ values are determined to 131 ± 10 µM for 6-PN, 119 ± 13 µM for 8-PN, 162 ± 46 µM for XN, and 59 ±15 µM for IX.

The present invention will be further illustrated by the following examples without being limited thereto.

### Examples

### Material and Methods

### Cell Culture

Caco-2 (human colorectal adenocarcinoma, ATCC HTB-37) cells (ATCC #HTB-37) were cultured in DMEM (Gibco #11965) supplemented with 10% Fetal Bovine Serum (HyClone #SH30071.03), 2 mM L-glutamine, 1% non-essential amino acids (NEAA) and 100 µg/mL penicillin/streptomycin (Gibco #15140), and were maintained at 37 °C and 5% CO₂.mL

### Infection Experiments

All experiments associated with SARS-CoV-2 were conducted in a Biosafety Level 3 laboratory. The recombinant infectious SARS-CoV-2 clone expressing mNeonGreen (icSARS-CoV-2-mNG) was obtained from the World Reference Center for Emerging Viruses and Arboviruses (WRCEVA) at the UTMB (University of Texas Medical Branch).

For testing antiviral activity, a total of 1 × 10⁴ Caco-2 cells were seeded in 96-well plates the day before infection in media containing 5% FCS. Caco-2 cells were infected with icSARS-CoV-2-mNG at a multiplicity of infection (MOI) = 0.26 or mock-infected. Simultaneously, cells were incubated with the either XN or IX at a concentration ranging from 100 to 3.125 µM at 37 °C. At 48 h post-infection (hpi), the cells were fixed with 2% paraformaldehyde (PFA) and stained with Hoechst (1µg/mL final concentration). Images were taken with Cytation3 (Biotek) and Hoechst+ and mNeonGreen+ cells were automatically counted by the Gen5 Software (Biotek). Infection rates were calculated as the ratio of Hoechst+ over mNeonGreen-positive cells. The IC₅₀ and cytotoxic CC₅₀ values (µM) were calculated using 4-parameter nonlinear regression (log(inhibitor) vs. response-variable slope) (GraphPad Prism 9.1.2).

### Cloning, expression, and purification of Sars-CoV-2-PLpro

The fusion of SARS-CoV-2 Plpro with the ubiquitin-like domain 2 (coding region 5132-5896 of SARS-CoV-2 genome, Genbank accession no OU652936.1), the N-terminal SUMO domain, and a hexa-histidine tag including an HRV 3C cleavage site by gene synthesis (Genscript) and cloned into pET15b. The resulting expression vector was transformed into *E. coli* BL21 (DE3). A single bacterial colony was used to inoculate 50 mL LB medium supplemented with 200 µg/mL ampicillin, 0.2% glucose and grown overnight. The overnight culture was spun down, resuspended in fresh medium and used to inoculate the 2 L of DYT supplemented with 50 mM Na₂HP₄, 0.2% glucose, 200 µg/mL ZnCl₂ and 200 µg/mL ampicillin. The culture was incubated at 37 °C until an OD₆₀₀ₙₘ = 0.6 was reached, then the temperature was reduced to 16 °C and expression of the transfected plasmid was induced by adding 0.2 mM IPTG. The cells were harvested after 48 h by centrifugation. Typically, 15 g wet weight cells could be recovered, which were then resuspended in 75 mL of ice-cold 20 mM Tris pH 8.0, 300 mM NaCl, 0.2 mM DTT, 1% glycerol, 1 mM MgCl₂. A spatula tip of lyophilized DNasel was added and cells were broken by 3-4 passages through an Emulsiflex (Avestin) at 4 °C and a pressure 1200 hPa. Cell debris and membranes were pelleted by ultracentrifugation at 100,000 g for 1 h and the resulting supernatant was filtered through 0.45 µm.

All subsequent chromatography steps were performed using Äkta purifier. The supernatant was applied to 10 mL column Talon Resin (Cytiva) equilibrated in resuspension buffer. After loading, the column was washed with the same buffer containing 5 mM imidazole until the absorption 280 nm reached baseline. PLpro was eluted applying a linear gradient of the same buffer containing 500 mM imidazole. The buffer was exchanged to 20 mM MES pH 6.0, 300 mM NaCl, 2 mM DTT, 1 mM MgCl₂, 5% glycerol, 1 mM MgCl₂ by passage over Nap10 column (Cytiva) equilibrated in the same buffer. Subsequently, 1 µg of HRV-3C protease per mg of fusion protein was added on ice. After 12 h, the buffer was changed via Nap10 column to 20 mM MES pH 6.0, 1% glycerol and the cleaved protein was loaded onto a 2 mL Source 15S column. Bound PLpro was eluted in a linear gradient with 20 mM MES pH 6.0, 1% glycerol, 500 mM NaCl. Protein fractions were analyzed by SDS-PAGE and fractions containing pure PLpro were combined, concentrated, and flash frozen in liquid N₂ until further use. Murine ISG15 modified at the C-terminus with propargylamine was prepared as known in the art.

### Reaction of PLpro with ISG15-PA probe

PLpro was diluted in 50 mM Na₂HPO₄, 500 mM NaCl and 10 mM DTT, pH 7.9, or in 50 mM Tris-HCI, 100 mM NaCl, 0.5 mg/mL 5 mM DTT, pH 7.6, to a final concentration of 5 µM. Pure ISG15-PA, was added at a 1:1 molar ratio and incubated for 1-10 minutes at room temperature or on ice.

### NanoBRET assay

HEK293T cells were cotransfected with HaloTag-Fusion construct (2 µg; Promega) and varying concentrations of Nluc-fusion vector (MDM2, 200 ng; RECQ1, 20 ng; Promega). After 20 h, cells were mixed with the HaloTag binding ligand 618 and replated 2 × 10⁴ cells per well on a white flat-bottom 96-well plate and incubated for 18-24 h. Luciferase substrate was added (Furimazine) to each well, and then Luminescence signal (counts/second) was measured using the Tecan Spark Machine. NanoBRET ratio was calculated by dividing the acceptor signal by the donor signal. Subsequently, the no-ligand control was subtracted from the ligand sample. Inhibitors were added at different concentrations.

### Characterization of SARS-CoV-2 PLpro by SAXS

SAXS measurements were performed on beamLine B21 at Diamond Light Source, Didcot, UK. Size-Exclusion Chromatography (SEC) coupled SAXS data were recorded using a Superdex 200 (3.2/300) column using 20 mM Tris, 100 mM NaCl, pH 6.5 as running buffer. A volume of 50 µl of each protein with a concentration of 11 mg/mL was injected onto the column. Buffer subtractions and all other subsequent analysis were performed with the program ScAtter (http://www.bioisis.net) or Chromixs. The SAXS curves were fitted with GNOM and 3D envelopes were reconstructed either with DENSS or with DAMMIF. For analysis with DENSS, the DENSS Web server (https://denss.ccr.buffalo.edu) was used running 20 individual DENSS reconstructions and subsequent alignment and averaging. For analysis with DAMMIF 13 independent reconstructions were aligned and averaged using DAMAVER. SUBCOMB and SITUS were used to align the 3D reconstructions and fit the X-ray structure of S100B dimer into the 3D envelopes. GNOM, DAMMIF, DAMAVER, and SUBCOMB are part of the ATSAS software suite. Figures were prepared with ScAtter and Chimera.

### Generation of ISGylated substrates and delSGylation assay

HEK293 cells were stimulated with 250 U/mL IFN-β for 24 h to induce ISGylation. Cells were lysed in 50 mM Tris-CI, pH 7.4,150 mM NaCl, 0.1 mM EDTA, and 1% Triton X-100, resuspended, and vortexed. The lysate was centrifuged at (4 °C) for 10 min at 14,000 g and the supernatant was used for further experiments. For the deISGylation assay the supernatants of the lysates were diluted in assay buffer (50 mM HEPES, pH 7.5, 150 mM NaCl, 0.1% BSA and freshly added 2.5 mM DTT) and the ISG15 cleavage reaction was started by addition of 2-4 µM PLpro to the lysates at 25 °C. Samples of the treated cell lysates with PLpro were taken after 1, 5, 15, 30, and 45 min and the reaction was immediately stopped by adding them to 5x SDS buffer supplemented with DTT and heating to 95 °C for 15 min. These samples as well as samples without PLpro were analyzed by Western blotting. Proteins were transferred to PVDF membrane and the membrane was subsequently blocked by incubation with PBS containing 5% BSA overnight. After blocking, the membrane was washed three times for 10 min in PBS-T and then incubated in 15 mL α-ISG15 antibody 1:2000 diluted in PBS supplemented with 3% BSA over night at 4 °C. After washing three times for 10 min in PBS-T, the membrane was incubated for 90 min at room temperature in 10 mL secondary antibody (a-rabbit-HRP antibody XXXX) 1:3000 diluted in PBS complemented by 1% milk powder and again washed four to five times for 10 min in PBS-T.

Developing took place in Image Quant LAS 500 with ECL reagent (BioRad). According to the manufactures protocol, equivalent volumes of 3 mL of the luminol/enhancer solution and the peroxide solution were mixed and given at the membranes for a few minutes.

### PLpro inhibition assays with hop compounds

Docking and following inhibition assays were done with the four hop compounds 6-PN, 8-PN, IX, and XN.

### Docking

Docking was performed with the program PLANTS (Protein-Ligand ANT System). The compounds were created in Marvin Sketch and exported in SMILES format. Coordinate files in pdb format were generated with ACEDRG. Input files for PLANTS in mol2 format were generated with the program SPORES. The docking results were visualized in PyMol (www.pymol.org).

### Kinetic assays and IC₅₀ determination

The activity of the SARS-CoV-2-PLpro was monitored either with the Z-Arg-Leu-Arg-Gly-Gly-AMC (Z-RLRGG-AMC; SEQ ID NO: 1) (Bachem), with ISG15-AMC (R&D systems) using, or with ISG15-rhodamine. Final concentrations for substrates were 5 µM for the peptide substrate (Z-RLRGG-AMC) and 100 nM for ISG15-AMC. All assays were performed as duplicates in 96 well plates with a final volume of 100 µl and a temperature of 20 °C in 50 mM HEPES, pH 7.5, 150 mM NaCl, 0.1 mg/mL BSA, and 2.5 mM DTT. The reaction was started by addition of PLpro and the linear increase in fluorescence was followed in a Tecan Infinite 200 PRO plate reader with excitation at 380 nm and recording the emission at 455 nm for 60 min. The specific activity of PLpro was calculated from the slope. A calibration curve was created from the fluorescence of a dilution series of AMC under the same conditions. Linear fits were calculated with OriginPro, Version 2020. (OriginLab Corporation, Northampton, MA, USA).

### Example 1:

### Infection Experiments

To investigate effects on viral replication of icSARS-CoV-2-mNG, Caco-2 cells were virus-infected and treated simultaneously with XN, IX, 6-PN, or 8-PN, for 48 h. XN exhibited the most pronounced inhibitory effect on virus replication with a half maximal inhibitory concentration (IC₅₀) of 3.28 µM and a cytotoxic concentration (CC₅₀) towards Caco-2 cells of 12.28 µM followed by 6-PN (IC₅₀ = 7.26 µM; CC₅₀ = 22.59 µM), IX (IC₅₀ = 20.11 µM; CC₅₀ = 19.49 µM), and 8-PN (IC₅₀ = 23.04 µM; CC₅₀ = 54.93 µM) (Figure 1 B,C). The therapeutic index (TI) is determined by dividing CC₅₀ with IC₅₀. Accordingly, XN showed the highest TI of 3.74 followed by 6-PN (3.11), 8-PN (2.38), and IX (0.97).

### Example 2:

### Docking calculations

To elucidate the underlying mechanism of hop compound induced viral replication inhibition, docking calculations were performed to identify the hop compound binding residues within PLpro, (Figure 2A). All hop compounds showed binding to the active site pocket of PLpro, thereby blocking the interaction with the C-terminus of ISG15. Comparison of the binding modes suggest that IX occupies a major part of the active site, binding with higher affinity to the enzyme than XN, 6-PN, and 8-PN.

### Example 3:

### Kinetic analysis

Hop compounds XN, 6-PN, 8-PN, and IX were tested for their potential to inhibit SARS-CoV-2 PLpro. The inhibition was characterized in an assay monitoring the cleavage of a peptide mimicking the C-terminus of human interferon-stimulated gene 15 (ISG15) that is cleaved by PLpro. Cleavage of the synthetic peptide releases the fluorophore AMC (7-Amino-4-methylcoumarin) resulting in an increase of fluorescence at 440 nm (Figure 2B) enabling determination of IC₅₀ values of PLpro inhibition. Calculated IC₅₀ values obtained for IX (59 ± 15 µM) revealed a much stronger inhibition of PLpro than IC₅₀ values for 6 PN, 8-PN, and XN (131 ± 10 µM, 119 ± 13 µM, and 162 ± 46 µM, respectively). These results are in good agreement with the results obtained from the in silico docking analysis (Figure 2A).

### Discussion

In the present invention, it could be shown that the hop compound XN is a very promising drug candidate to combat COVID-19 infection. Of particular interest in this context is the ability of an active ingredient to inhibit the replication of SARS-CoV-2, which could be demonstrated very well for XN by using the fluorescently labeled SASRS-CoV-2 wild-type virus strain icSARS-CoV-2-mNG in Caco-2 cells in cell culture experiments. Fluorescent labeling allowed to quantify the change in viral replication fluorometrically but also microscopically. The IC₅₀ of XN was 3.28 µM with a CC₅₀ of 12.28 µM and XN therefore showed the highest TI of 3.74 of all hop compounds tested (6-PN: 3.11; 8-PN: 2.38; IX: 0.97). It should be emphasized that Caco-2 cells are malignant cells, even though they represent a much-used model system for human transport processes. Since prenylated flavonoids from hops also exhibit pronounced antitumoral effects, it can be speculated that the TI when using healthy human cells would actually be even higher than measured in this study. To identify one possible mechanism of action, the effect of the four hop compounds on PLpro was investigated. The compounds all inhibited PLpro but XN showed the lowest inhibitory effect here (*in silico*

## Claims

1. A composition comprising xanthohumol (XN) for use in a method of preventing or treating a Coronavirus infection in a subject.

2. The composition for use according to claim 1, wherein the Coronavirus is a Betacoronavirus.

3. The composition for use according to claim 1 or claim 2, wherein the Coronavirus is selected from the group consisting of Betacoronavirus 1 (Bovine Coronavirus, Human coronavirus OC43), Hedgehog coronavirus 1, Human coronavirus HKU1, Middle East respiratory syndrome (MERS)-related coronavirus (MERS-CoV), Murine coronavirus, *Pipistrellus* bat coronavirus HKU5, *Rousettus* bat coronavirus HKU9, Severe acute respiratory syndrome (SARS)-related coronavirus (SARS-CoV, SARS-CoV-2), and *Tylonycteris* bat coronavirus HKU4.

4. The composition for use according to any one of claims 1 to 3, wherein the Coronavirus is MERS-CoV, SARS-CoV or SARS-CoV-2.

5. The composition for use according to any one of claims 1 to 4, wherein the Coronavirus is SARS-CoV-2.

6. The composition for use according to any one of claims 1 to 5, wherein the subject is a bird or a mammal.

7. The composition for use according to any one of claims 1 to 6, wherein the subject is a mammal.

8. The composition for use according to any one of claims 1 to 7, wherein the subject is a feline, canine, bovine, swine, equine, camel, murine, mustelid, or human.

9. The composition for use according to any one of claims 1 to 8, wherein the subject is a human.

10. The composition for use according to any one of claims 1 to 9, wherein the composition comprises one or more additional agents, selected from the group consisting of polyphenols, antioxidants, minerals, anti-inflammatory agents and antiviral agents.

11. The composition according to any one of claims 1 to 10 for use in a method of treating a Coronavirus infection in a subject.

12. The composition for use according to any one of claims 1 to 11, wherein the composition is administered to the subject in a daily dose of 0.05 to 100 mg XN.

13. The composition for use according to any one of claims 1 to 11, wherein the composition is administered to the subject in a dose of 0.625 µg to 1250 µg XN per kg body weight of the subject per day (0.625 to 1250 µg/kg/d XN).

14. The composition for use according to any one of claims 1 to 13, wherein the composition is administered to the subject orally or intranasally.

15. The composition for use according to any one of claims 1 to 14, wherein the composition is in the form of tablets, capsules, lozenges, sachets, powders, drops, solution, spray, dosage spray, nasal spray, nasal drops, or nasal cream, or wherein the composition is in the form of a food supplement or dietary supplement.
